(19) Europäisches Patentamt
European Patent Office
Office européen des brevets

(11) **EP 2 568 292 A1**

(12) # EUROPÄISCHE PATENTANMELDUNG

(43) Veröffentlichungstag:
**13.03.2013 Patentblatt 2013/11**

(51) Int Cl.:
***G01N 33/68*** *(2006.01)*

(21) Anmeldenummer: **12180531.1**

(22) Anmeldetag: **15.08.2012**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**

(30) Priorität: **09.09.2011 DE 102011082432**

(71) Anmelder: **Henkel AG & Co. KGaA
40589 Düsseldorf (DE)**

(72) Erfinder: **Giesen, Melanie
47608 Geldern (DE)**

(54) **Verfahren zur Altersbestimmung behaarter Säugetiere**

(57) Ein Verfahren, das eine Beurteilung des Alters behaarter Säugetiere ermöglicht und in Abhängigkeit vom Ergebnis Vorschläge für Produkte unterbreiten und Werbeaussagen für Produkte stützen kann, umfaßt die Schritte, daß man

a) dem Säugetier eine definierte Menge M an Haaren entnimmt,

b) die Haare einem partiellen Abbau unterwirft,

c) das/die Abbauprodukt(e) auf Art und Menge von Peptidfragmenten untersucht und

d) anhand der Menge definierter Fragmente das Alter des Säugetiers ermittelt.

EP 2 568 292 A1

**Beschreibung**

Die Erfindung betrifft ein Verfahren zur Altersbestimmung behaarter Säugetiere, das eine Altersbestimmung, eine gezielte kosmetische Behandlung und fundierte Werbeaussagen ermöglicht.

Gepflegtes, volles Haar ist ein Verbraucherbedürfnis über alle Kulturen und Altersgrenzen hinweg. Eine schier unendliche Vielfalt haarkosmetischer Produkte will dieses Verbraucherbedürfnis befriedigen, allerdings führt diese Vielfalt auch zu einer Verunsicherung der Verbraucher, welches Produkt für sein Haar und sein gewünschtes Aussehen am besten geeignet ist.

Zudem wünscht der Verbraucher, ein "jüngeres" Aussehen. Entsprechende Aussagen in der Produktwerbung werden allerdings mit Skepsis wahrgenommen.

Es besteht daher der Bedarf, das Alter eines Säugetiers durch eine Haaranalyse treffsicher feststellen zu können, unabhängig davon, ob es sich um einen Menschen, oder z.B. einen Hund oder eine Katze handelt. Anhand der Altersbestimmung kann dann ein kosmetisches Mittel appliziert und nach geeigneter Behandlungszeit eine erneute Altersbestimmung durchgeführt werden. Bei erfolgreicher Behandlung ist dann die Aussage "um x Jahre verjüngtes Haar" fundiert abgesichert.

Weiter besteht das Bedürfnis, dem Verbraucher jenseits von Marketingversprechen die Auswahl eines speziell auf sein Haar abgestimmten Produktes zu ermöglichen. Bislang sind Untersuchungsmethoden am Individuum der medizinischen Forschung vorbehalten, weil sie entweder zu aufwändig und damit zu teuer sind oder zu viel Probesubstanz (= Haar) benötigen. Eine individuelle, zuverlässige und auch jenseits der Medizin anwendbare Methode, das Haar eines Konsumenten zu beurteilen und anhand der Beurteilung gezielt Produkte anzubieten, existiert im kosmetischen Massenmarkt nicht.

Der vorliegenden Erfindung lag daher die Aufgabe zugrunde, ein Verfahren bereitzustellen, das eine Beurteilung des Alters behaarter Säugetiere ermöglicht und in Abhängigkeit vom Ergebnis Vorschläge für Produkte unterbreiten und Werbeaussagen für Produkte stützen kann.

Gegenstand der vorliegenden Erfindung ist in einer ersten Ausführungsform ein Verfahren zur Altersbestimmung behaarter Säugetiere, bei dem man

a) dem Säugetier eine definierte Menge M an Haaren entnimmt,
b) die Haare einem partiellen Abbau unterwirft,
c) das/die Abbauprodukt(e) auf Art und Menge von Peptidfragmenten untersucht und
d) anhand der Menge definierter Fragmente das Alter des Säugetiers ermittelt.

In einem ersten Schritt des erfindungsgemäßen Verfahrens wird dem Säugetier eine definierte Menge M an Haaren entnommen. Die Entnahme kann beispielsweise im Rahmen eines regulären Friseurbesuches erfolgen, sie kann aber auch in "Heimanwendung" vom Kunden selbst erfolgen, der die Haare anschließend verpackt und an ein geeignetes Labor sendet. Auf diese Weise können Probenentnahme und -Untersuchung räumlich und zeitlich entkoppelt werden, auch eine Übersendung der Proben durch den Kunden selbst kann auf diese Weise erfolgen.

Da es im Rahmen des erfindungsgemäßen Verfahrens auf die Untersuchung nicht belebter Materie ankommt, kann auf das unangenehme Zupfen aus behaarter Haut unter Erhalt des Haarfollikels verzichtet werden. Vielmehr genügen einzelne Haare, die entweder abgeschnitten oder aus einer Bürste gewonnen werden können.

Die definierte Menge M soll sicherstellen, daß auch weniger häufig auftretende Abbauprodukte detektiert werden können. Da das Ergebnis des erfindungsgemäßen Verfahrens von der prozentualen Verteilung der einzelnen Fragmente abhängt, ist die Menge variabel. Sie liegt bei erfindungsgemäß bevorzugten verfahren zwischen 10 μg und 10 g, vorzugsweise zwischen 100 μg und 1 g, besonders bevorzugt zwischen 250 μg und 500 μg.

Im zweiten Schritt des erfindungsgemäßen Verfahrens werden die Haare einem partiellen Abbau unterworfen. Um in diesem Schritt nachvollziehbare Aussagen zu erhalten, müssen alle Proben derselben Behandlung unterworfen werden. Je nach Wahl der Parameter (Abbaugreagentien, Druck, Temperatur, Zeit usw.) existiert eine große Vielzahl von Varianten des erfindungsgemäßen Verfahrens. verläßliche und nachvollziehbare Aussagen lassen sich dabei nur dann treffen, wenn immer dieselbe Verfahrensvariante mit denselben Parametern benutzt wird.

Die Verläßlichkeit der Methode kann durch Kombination mehrerer erfindungsgemäßer Verfahrensvarianten gesteigert werden. So kann z.B. eine saure Hydrolyse einmal bei Umgebungsbedingungen (atmosphärischer Druck, 20°C) durchgeführt werden, ein zweites Mal bei erhöhter Temperatur und unter Druck. Die jeweils unterschiedlichen Fragmentprofile sind für das jeweilige Alter des Säugetiers, dessen Haare untersucht wurden, spezifisch, und die Genauigkeit der Methode wird durch die Kombination von Verfahrensvarianten erhöht.

Grundsätzlich läßt sich der partielle Abbau in Schritt b) des erfindungsgemäßen Verfahrens auf unterschiedliche Art und Weise realisieren. Die Zerlegung der Keratine in Proteinfragmente kann durch verschiedene Methoden (z.B. Hydrolyse, Extraktion oder enzymatischen Verdau) erfolgen.

Bevorzugte erfindungsgemäße Verfahren sind **dadurch gekennzeichnet, daß** die Haare in Schritt b) einer Hydrolyse, vorzugsweise einer Hydrolyse mittels konzentrierter Salzsäure unterworfen werden.

Besonders bevorzugt ist hierbei eine Hydrolyse der Keratinfasern in konzentrierter Salzsäure bei 20°C und 1013,25 mbar für zwei Stunden.

Alternativ können die Haarkeratine durch zersetzende Extraktion in Proteinfragmente gespalten werden. Hier sind bevorzugte erfindungsgemäße Verfahren **dadurch gekennzeichnet, daß** die Haare in Schritt b) einer zersetzenden Extraktion mittels einer Tensidlösung, vorzugsweise einer Extraktion mit einer Lösung von Natriumdodecylsulfat und/oder ethoxylierter Nonylphenole, besonders bevorzugt einer Extraktion mit einer Lösung von Harnstoff und Natriumdodecylsulfat und/oder ethoxylierter Nonylphenole unterworfen werden.

Auch hier lassen sich unzählige Untervarianten des erfindungsgemäßen Verfahrens durch Wahl des Tensids bzw. der Tensidmischung, die Konzentration des Tensids, die Temperatur, den Druck und die Einwirkzeit realisieren.

Alternativ können die Haarkeratine durch enzymatischen Abbau in Proteinfragmente gespalten werden. Der enzymatische Abbau kann z.B. durch Trypsin erfolgen. Trypsin hydrolysiert spezifisch Peptidbindungen an der Carboxylseite von Arginin oder Lysinresten. Hier sind bevorzugte erfindungsgemäße Verfahren **dadurch gekennzeichnet, daß** die Haare in Schritt b) einem enzymatischen Abbau, vorzugsweise einem Abbau durch Trypsin unterworfen werden.

In verschiedenen Fällen ist es vorteilhaft, verschiedene Methoden miteinander zu kombinieren. So kann einer salzsaure Hydrolyse in einem zweiten Schritt ein enzymatischer Abbau folgen.

Im dritten Schritt des erfindungsgemäßen Verfahrens wird/werden das/die Abbauprodukt(e) auf Art und Menge von Peptidfragmenten untersucht. Dazu wird die Mischung der Abbauprodukte in geeigneter Weise getrennt und detektiert. Aus der Verteilung und Menge einzelner oder aller Abbauprodukte können dann Rückschlüsse auf das Alter des Säugetiers geschlossen werden, das Träger der Haare war. Dabei kann je nach Abbaumethode in Schritt b) ein einzelnes Abbauprodukt als Indikationsgröße gewählt werden, vorteilhafterweise wählt man aber mehrere unterschiedliche Abbauprodukte als Leitgrößen, um die Signifikanz des erfindungsgemäßen Verfahrens zu erhöhen.

Beispielsweise kann nach einem definierten Abbauschritt b) die prozentuale Menge eines bestimmten Oligo- oder Polypeptids gemessen werden. Der prozentuale Anteil dieses Peptids an der Gesamtmenge der Abbauprodukte variiert mit dem Alters des Säugetiers, so daß mit Hilfe einer vorher erstellten "Eichkurve" das Alter des Säugetiers bestimmt werden kann

Die einzelnen Detektionsverfahren für Schritt c) des erfindungsgemäßen Verfahrens werden nachfolgend beschrieben:

In erfindungsgemäß bevorzugten Verfahren werden das/die Abbauprodukt(e) in Schritt c) gelelektrophoretisch auf Art und Menge von Peptidfragmenten untersucht, wobei die zweidimensionale Gelelektrophorese bevorzugt ist.

Bei der Gelelektrophorese wandert eine Mischung aus zu trennenden Molekülen unter Einfluss eines elektrischen Felds durch ein Gel, welches in einer ionischen Pufferlösung liegt. Je nach Größe und Ladung der Moleküle bewegen sich diese unterschiedlich schnell durch das als Molekularsieb wirkende Gel. Dabei wandern kleine, negativ geladene Moleküle am schnellsten in Richtung der positiv geladenen Anode und positiv geladene Moleküle in Richtung der negativ geladenen Kathode.

Die Moleküle des Gels, beispielsweise Agarose oder Polyacrylamid, bilden ein engmaschiges Netz, das die zu trennenden Moleküle bei ihrer Wanderung im elektrischen Feld behindert.

Agarose-Gele sind relativ großporig (150 nm bei einprozentigen, 500 nm bei 0,16-prozentigen Gelen) und eignen sich gut zur Trennung von DNA und hochmolekularen Proteinen. Polyacrylamid-Gele weisen wesentlich kleinere Poren auf (3-6 nm). Die Porengröße hängt von der Acrylamidkonzentration und dem Vernetzungsgrad ab. Je nach Anwendung werden dem Gel verschiedene Zusatzstoffe zugesetzt, beispielsweise SDS bei der SDS-Polyacrylamidgelelektrophorese (SDS-PAGE) oder Ampholyte bei der Isoelektrischen Fokussierung (IEF). SDS denaturiert und entfaltet Proteine, so dass sie, von SDS eingehüllt, kettenförmig gelöst schwimmen. Die Ladungsdichte solcher Ketten ist, unabhängig von ihrer Länge, ungefähr gleich. Aus diesem Grunde haben die SDS-Protein-Komplexe in freier Lösung, d. h. ohne die zusätzliche Einwirkung von Gelen, gleiche Wanderungsgeschwindigkeit. Es ist aber auch nicht unüblich, auf das SDS zu verzichten, wenn den Proben schon vor der Auftragung SDS im Probenpuffer zugegeben wird.

Die klassische Gelelektrophorese wird als Zonenelektrophorese durchgeführt. Eine Methode zur Erzielung einer höheren Auflösung ist die diskontinuierliche Elektrophorese.

Bei der Gelelektrophorese entsteht Wärme. Diese muss abgeführt werden, um optimale Bedingungen zu gewährleisten. Deswegen sollte die Gelelektrophorese in gekühlten Apparaturen bei konstanten Temperaturen durchgeführt werden, um reproduzierbare Ergebnisse zu erzielen.

Im Idealfall wird die Elektrophorese beendet, wenn die kleinsten beziehungsweise mobilsten Moleküle das Ende des Gels erreicht haben. Das garantiert die höchstmögliche Auftrennung der Moleküle.

Zur Auswertung des Gels nach der Elektrophorese werden die zu trennenden Moleküle entweder vor der Elektrophorese radioaktiv markiert und anschließend in einer Autoradiographie nachgewiesen oder nach der Elektrophorese mit verschiedenen Farbstoffen wie beispielsweise Ethidiumbromid "gefärbt" und unter UV-Licht betrachtet. So verfährt man etwa mit DNA-Fragmenten. Proteine können angefärbt werden (Färbung nach Fairbanks, Silberfärbung) und/oder immunologisch nachgewiesen werden (Western Blot). Gleiche Moleküle laufen in diskreten Zonen - umgangssprachlich als *Banden* bezeichnet - durch das Gel. Mehrere Proben können parallel nebeneinander gleichzeitig durch dasselbe Gel laufen. Ist die Größe einiger Moleküle bekannt, kann man durch Vergleich von deren Banden mit den restlichen Banden die Größe der anderen Moleküle abschätzen. Solche Molekülmassenstandards sind kommerziell erhältlich. Ähnlich funktioniert auch ein Comigrationsstandard, mit dessen Hilfe eine unbekannte mit einer bekannten Probenzusammensetzung verglichen wird. Als Molekülmassenstandards werden DNA (Desoxyribonukleinsäure) oder Proteine verwendet.

Eine Bestimmung der Menge einer Substanz in einer Bande beziehungsweise der relative Anteil einer Bande ist nach der Färbung des Gels und einer anschließenden densitometrischen Auswertung möglich. Zur Bestimmung der Messwerte eines Geles wie z. B. Laufweiten, Molekülmassen, Quantifizierungen oder Normalisierung wird in den meisten Fällen eine Auswertungssoftware genutzt.

Die 2D-Gelelektrophorese wird beispielsweise in L.D. Adams, Two-dimensional Gel Electrophoresis using the Isodalt System oder in L.D. Adams & S.R. Gallagher, Two-dimensional Gel Electrophoresis using the O'Farrell System; beide in Current Protocols in Molecular Biology (1997, Eds. F.M. Ausubel et al.), Unit 10.3.1 - 10.4.13; oder in 2-D Electrophoresis-Manual; T. Berkelman, T. Senstedt; Amersham Pharmacia Biotech, 1998 (Bestell-Nr. 80-6429-60), beschrieben.

In erfindungsgemäß ebenfalls bevorzugten Verfahren werden das/die Abbauprodukt(e) in Schritt c) flüssigchromatographisch auf Art und Menge von Peptidfragmenten untersucht, wobei die LC bevorzugt ist.

Die Flüssigchromatographie ist als Spezialgebiet der Chromatographie eine physikalische Trennmethode. Als mobile Phase dient eine Flüssigkeit. Sie kann entweder in einer Säule oder einer Ebene erfolgen. Moderne Methoden zeichnen sich hierbei durch sehr kleine Partikelgrößen und hohe Einstromdrücke aus. Man unterscheidet:

- Papierchromatographie,
- Dünnschichtchromatographie (kurz DC) und
- Säulenchromatographie

    ○ Niederdruckflüssigkeitschromatographie und
    ○ Hochleistungsflüssigkeitschromatographie (kurz HPLC)
    ○ Gel-Permeations-Chromatographie (GPC)
    ○ Superkritische Flüssigchromatographie (SFC)

- Feld-Fluss-Fraktionierung (kurz FFF)

    ○ Fluss-Feld-Fluss-Fraktionierung

        ■ Symmetrische Fluss-Feld-Fluss-Fraktionierung (kurz SF4)

        ■ Asymmetrische Fluss-Feld-Fluss-Fraktionierung (kurz AF4)

    ○ Elektrische Feld-Fluss-Fraktionierung
    ○ Gravitations-Feld-Fluss-Fraktionierung

Dabei unterscheidet man offene Systeme (z. B. Dünnschichtchromatographie, Papierchromatographie), wobei die stationäre Phase herausgenommen und direkt untersucht werden kann, und geschlossene Systeme (z. B. Säulenchromatographie, HPLC). Bei geschlossenen Systemen werden die Substanzen nach Elution mit einem Lösungsmittel isoliert oder mit einem Detektor angezeigt.

Ob diese Trennmethode zur Trennung eines Substanzgemisches eingesetzt werden kann, hängt vor allem davon ab, ob alle Substanzen des Gemisches in der Mobilen Phase gelöst werden können und ob es eine stationäre Phase gibt, die eine ausreichende Selektivität zwischen den Substanzen aufweist. Durch Elution oder Kapillarwirkung des Lösungsmittels kommt es durch die stationäre Phase zu einem Sortierprozess entsprechend den Moleküleigenschaften. Durch das nachlaufende Lösungsmittel bilden sich Substanzzonen aus, die am unteren Rohrausgang aufgefangen werden können.

Als stationäre Phasen kommen häufig modifizierte und nicht modifizierte Silikagele und Polymere, z. B. Polystyrol-Divinylbenzol-Copolymere (PS/DVB), zum Einsatz. In der Feld-Fluss-Fraktionierung werden verschiedene Trennfelder (Flussfelder, thermische Felder, etc.) als stationäre Phase eingesetzt.

In erfindungsgemäß ebenfalls bevorzugten Verfahren werden das/die Abbauprodukt(e) in Schritt c) massenspektrometrisch auf Art und Menge von Peptidfragmenten untersucht.

Die massenspektrometrische Charakterisierung der Proteine oder Proteinfragmente erfolgt in der Fachwelt bekannter Weise, beispielsweise wie in den folgenden Literaturstellen beschrieben:

Methods in Molecular Biology, 1999; Vol 112; 2-D Proteome Analysis Protocols; Editor: A. J. Link; Humana Press; Totowa; New Jersey. Darin insbesondere: Courchesne, P. L. und Patterson, S. D.; S. 487-512.

Carr, S. A. und Annan, R. S.; 1997; in: Current Protocols in Molecular Biology; Editor: Ausubel, F. M. et al.; John Wiley and Sons, Inc. 10.2.1-10.21.27.

Bei der Massenspektrometrie muß der zu untersuchende Analyt zunächst ionisiert werden, um dann in geeigneter Weise detektiert zu werden. Als Ionisationsmethoden kommen Atmosphärendruck-Methoden (Elektrospray (ESI), Desorptions Elektrospray (DESI), Chemische Ionisation bei Atmosphärendruck (APCI), Photoionisation bei Atmosphärendruck (APPI), Laserionisation bei Atmosphärendruck (APLI), Ionisation durch induktiv gekoppeltes Plasma (ICP), Direct Analysis in Real Time (DART)) oder Niederdruck-Methoden (Elektronenstoßionisation (EI), Chemische Ionisation (CI), Matrix-unterstützte Laser-Desorption/Ionisation (MALDI), Electron Capture Dissociation (ECD), Fast Atom Bombardment (FAB), Felddesorption (FD), Feldionisation (FI), Photoionisation (PI)) in Frage.

Im Hinblick auf die größeren Peptidfragmente, die im erfindungsgemäßen Verfahren zu untersuchen sind, hat sich die Ionisation mittels Matrix-unterstützter Laser-Desorption/Ionisation *(MALDI)* besonders bewährt. MALDI ist ein Verfahren zur Ionisation von Molekülen, das sich seit seiner Entwicklung in den 1980er-Jahren als besonders effektiv für die Massenspektrometrie von großen Molekülen und Polymeren sowie Biopolymeren (z. B. Proteinen) erwiesen hat.

Die Detektion erfolgt bei Ionisation mittels MALDI überwiegend über die Flugzeitanalyse. MALDI-TOF ist demnach ein Verfahren zur Massenanalyse von chemischen Verbindungen. Es ist eine Kombination aus den Verfahren Matrix-unterstützte Laser-Desorption/Ionisation (MALDI) und der Flugzeitanalyse (engl. time of flight, TOF)

Bei MALDI ionisiert man den Analyten mittels Laserbeschuss (meist durch Ultraviolettstrahlung bei 337 nm, es finden aber auch zunehmend Anregungen durch Infrarotstrahlung Verwendung) über eine Matrix, in welche die eigentlichen Analyten eingebettet sind. Dazu wird der zu untersuchende Analyt und die Matrix (meist ein Benzoesäurederivat wie 2,5-Dihydroxybenzoesäure (DHB) oder auch ein Zimtsäurederivat) auf einem metallischen Träger cokristallisiert. Durch Laserbeschuss verdampft die Matrix explosionsartig und die zu untersuchenden Analytmoleküle werden mitgerissen. Dabei findet üblicherweise gleichzeitig eine Ionisierung des Analyten statt. Die entstandenen Ionen werden in einem elektrischen Feld beschleunigt. Typische Beschleunigungsspannungen hierfür liegen bei 10-30 kV. Wegen der gepulsten Ionenerzeugung bietet sich für die Massenanalyse ein TOF-Analysator (Tof-Massenspektrometer) an.

Hierbei gilt für die Flugzeit in Abhängigkeit von Masse *(m)* und Ladungszahl (z) folgender Zusammenhang:

$$tof \propto \sqrt{\frac{m}{z}}$$

Ein Ionendetektor (oft ein Sekundärelektronenvervielfacher wie etwa eine Mikrokanalplatte) wandelt die ankommenden Ionen in ein elektrisches Signal um. Der Vorteil dieser Messmethode ist die schnelle Analyse eines vergleichsweise großen Massenbereichs. Bemerkenswert ist, dass der TOF-Analysator keine prinzipielle Massenbegrenzung zu großen Massen hin besitzt, so dass schon Ionen von m/z bis zu 1.000.000 gemessen wurden. Als ionisierte Spezies treten vor allem die Pseudomolekülionen [M+H]+, [M+Na]+ oder auch [M+K]+ auf. Das Auftreten (bzw. die relativen Intensitäten) der unterschiedlichen Spezies hängt jedoch stark von der Vorgeschichte der Probe sowie von der Substanzklasse an sich ab. Wie bereits erwähnt, kann je nach Abbaumethode in Schritt b) ein einzelnes Abbauprodukt als Indikationsgröße gewählt werden, vorteilhafterweise wählt man aber mehrere unterschiedliche Abbauprodukte als Leitgrößen, um die Signifikanz des erfindungsgemäßen Verfahrens zu erhöhen.

Erfindungsgemäße Verfahren, bei denen ein, zwei oder drei Leitfragmente definiert und deren Menge zur Altersbestimmung genutzt wird, sind demnach bevorzugt.

Nach einem definierten Abbauschritt b) kann die prozentuale Menge eines bestimmten Oligo- oder Polypeptids gemessen werden. Der prozentuale Anteil dieses Peptids an der Gesamtmenge der Abbauprodukte variiert mit dem Alters des Säugetiers, so daß mit Hilfe einer vorher erstellten "Eichkurve" das Alter des Säugetiers bestimmt werden kann. Entsprechend sind erfindungsgemäße Verfahren, bei denen die Art und Menge definierter Peptidfragmente von Säugetieren definierten Alters bestimmt wird und als Referenzwert für die Altersbestimmung dient, bevorzugte Ausführungsformen der vorliegenden Erfindung.

Besonders bevorzugte erfindungsgemäße Verfahren nutzen dabei Referenzkurven "definiertes Peptid vs. Alter" zur Altersbestimmung.

Die "Sollwerte" dieser Referenzkurven lassen sich beispielsweise durch die Untersuchung gesunder Haare ermitteln, wobei bevorzugt Mittelwerte aus einem Kollektiv gebildet werden. Besonders bevorzugt werden die Kollektive dabei besonders einheitlich und besonders groß gebildet, d.h. der "Basiswert" wird aus den Haaren möglichst vieler Individuen ermittelt, die möglichst viele Eigenschaften miteinander teilen, wobei dem Alter und dem Geschlecht die größte Bedeutung zukommt. In erfindungsgemäß bevorzugten Verfahren führt man die Erhebung der Sollwerte in Schritt c) mit einer in Bezug auf das Alter und das Geschlecht ausreichend großen (vorzugsweise mindestens 10, besonders bevorzugt mindestens 50 und insbesondere mindestens 100 Personen) Kontrollgruppe durch.

Ganz besonders bevorzugt werden noch weitere Spezifizierungen/Gruppierungen eingeführt und die Grenzen möglichst eng gesteckt. So könnte eine Kontrollgruppe "weiblich, drittes Lebensjahrzehnt" weiter aufgeteilt werden in "weiblich, 25 Jahre" oder "weiblich, Geburtsjahrgang xxxx" und gegebenenfalls noch weiter in "weiblich, 25 Jahre, blond, dünnes Haar" usw..

Durch geeignete Wahl der Kontrollgruppe und der in dieser Kontrollgruppe ermittelten Werte für die einzelnen Abbauprodukte Faktoren kann die Abweichung des Meßergebnisses ermittelt werden. Wird nicht nur ein Abbauprodukt bestimmt, sondern eine Mehrzahl dieser Produkte, kann das Alters sehr treffsicher ermittelt werden.

Auf dieser Datenlage kann dann das Haar des Säugetiers mit kosmetischen Mitteln behandelt werden. Im Anschluß an eine solche Behandlung kann mit dem erfindungsgemäßen Verfahren erneut das Alter bestimmt werden, wobei sich die Wirksamkeit des kosmetischen Produktes an der "Verjüngung" des Haares messen läßt. Auf diese Weise lassen sich auch Werbeaussagen wir "um 10 Jahre jüngeres Haar" stützen.

Zudem kann der Erfolg des jeweiligen Kosmetikums durch eine weitere Analyse nach einer bestimmten Behandlungszeit aufgezeigt und dokumentiert werden. Dem individuellen Zufriedenheitsgefühl des Verbrauchers wird damit eine objektive Analyse zur Seite gestellt, die geeignet ist, dem Kunden an eine Herstellfirma und ihre Produkte zu binden.

**Patentansprüche**

1.  Verfahren zur Altersbestimmung behaarter Säugetiere, **dadurch gekennzeichnet, daß** man

    a) dem Säugetier eine definierte Menge M an Haaren entnimmt,
    b) die Haare einem partiellen Abbau unterwirft,
    c) das/die Abbauprodukt(e) auf Art und Menge von Peptidfragmenten untersucht und
    d) anhand der Menge definierter Fragmente das Alter des Säugetiers ermittelt.

2.  Verfahren nach Anspruch 1, **dadurch gekennzeichnet, daß** die Haare in Schritt b) einer Hydrolyse, vorzugsweise einer Hydrolyse mittels konzentrierter Salzsäure unterworfen werden.

3.  Verfahren nach einem der Ansprüche 1 oder 2, **dadurch gekennzeichnet, daß** die Haare in Schritt b) einer zersetzenden Extraktion mittels einer Tensidlösung, vorzugsweise einer Extraktion mit einer Lösung von Natriumdodecylsulfat und/oder ethoxylierter Nonylphenole, besonders bevorzugt einer Extraktion mit einer Lösung von Harnstoff und Natriumdodecylsulfat und/oder ethoxylierter Nonylphenole unterworfen werden.

4.  Verfahren nach einem der Ansprüche 1 bis 3, **dadurch gekennzeichnet, daß** die Haare in Schritt b) einem enzymatischen Abbau, vorzugsweise einem Abbau durch Trypsin unterworfen werden.

5.  Verfahren nach einem der Ansprüche 1 bis 4, **dadurch gekennzeichnet, daß** das/die Abbauprodukt (e) in Schritt c) gelelektrophoretisch auf Art und Menge von Peptidfragmenten untersucht werden, wobei die zweidimensionale Gelelektrophorese bevorzugt ist.

6.  Verfahren nach einem der Ansprüche 1 bis 5, **dadurch gekennzeichnet, daß** das/die Abbauprodukt (e) in Schritt c) flüssigchromatographisch auf Art und Menge von Peptidfragmenten untersucht werden, wobei die LC bevorzugt ist.

7.  Verfahren nach einem der Ansprüche 1 bis 6, **dadurch gekennzeichnet, daß** das/die Abbauprodukt (e) in Schritt c) massenspektrometrisch auf Art und Menge von Peptidfragmenten untersucht werden.

8.  Verfahren nach einem der Ansprüche 1 bis 7, **dadurch gekennzeichnet, daß** ein, zwei oder drei Leitfragmente definiert und deren Menge zur Altersbestimmung genutzt wird.

9.  Verfahren nach Anspruch 1 bis 8, **dadurch gekennzeichnet, daß** die Art und Menge definierter Peptidfragmente von Säugetieren definierten Alters bestimmt wird und als Referenzwert für die Altersbestimmung dient.

10. Verfahren nach Anspruch 9, **dadurch gekennzeichnet, daß** Referenzkurven "definiertes Peptid vs. Alter" erzeugt und zur Altersbestimmung genutzt werden.

Europäisches Patentamt
European Patent Office
Office européen des brevets

## EUROPÄISCHER RECHERCHENBERICHT

Nummer der Anmeldung

EP 12 18 0531

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | MELANIE GIESEN ET AL: "Ageing processes influence keratin and KAP expression in human hair follicles", EXPERIMENTAL DERMATOLOGY, Bd. 20, Nr. 9, 1. September 2011 (2011-09-01), Seiten 759-761, XP055033560, ISSN: 0906-6705, DOI: 10.1111/j.1600-0625.2011.01301.x | 1,5,7,8 | INV. G01N33/68 |
| Y | * Kapitel "conclusion" * ----- | 1-10 | |
| Y | Clarence R Robbins: "Chemical Composition" In: "Chemical and Physical Behavior of Human Hair", 19. Juli 2009 (2009-07-19), Springer, XP055040369, ISBN: 978-0-38-795094-5 Seiten 63-104, * Kapitel 2 * ----- | 1-10 | |

RECHERCHIERTE SACHGEBIETE (IPC)

G01N

Der vorliegende Recherchenbericht wurde für alle Patentansprüche erstellt

| Recherchenort | Abschlußdatum der Recherche | Prüfer |
|---|---|---|
| Den Haag | 9. Oktober 2012 | Rosin, Oliver |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes
   Dokument

EPO FORM 1503 03.82 (P04C03)

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- Current Protocols in Molecular Biology. 1997, 10.3.1-10.4.13
- **T. BERKELMAN ; T. SENSTEDT.** 2-D Electrophoresis-Manual. 1998
- Methods in Molecular Biology. **COURCHESNE, P. L. ; PATTERSON, S. D.** 2-D Proteome Analysis Protocols. Humana Press, 1999, vol. 112, 487-512
- **CARR, S. A. ; ANNAN, R. S. et al.** Current Protocols in Molecular Biology. John Wiley and Sons, Inc, 1997, 10.2.1-10.21.27